# EUROPEAN PATENT APPLICATION

(11) **EP 3 540 046 A1**
(43) Date of publication of application: **18.09.2019**
(21) Application number: 18161619.4
(22) Date of filing: 13.03.2018
(51) Int. Cl.: C12N 5/077, C12N 5/071

(54) **PROCESS FOR PRODUCING CARDIAC ORGANOIDS**

(71) Applicant: Medizinische Hochschule Hannover, 30625 Hannover (DE)
(72) Inventor: Zweigerdt, Robert, 30625 Hannover (DE); Drakhlis, Lika, 30625 Hannover (DE)
(74) Representative: Taruttis, Stefan Georg

(57) **Abstract**

The invention provides an in vitro process for producing a cardiac organoid from cultivated pluripotent stem cells, which cardiac organoids reproducibly have a structure of specific cardiac cell layers.

## Description

The present invention relates to an in vitro process for producing cardiac organoids using pluripotent stem cells (PSC), preferably human PSC, to the cardiac organoids obtainable by the process, and to a process for analysing the activity of a test compound on cardiac cells by exposing the PSC during the process for their production to the test compound and/or exposing at least one cardiac organoid obtainable by the process for their production to the test compound, and analysing the organoid, e.g. in comparison to PSC and/or a cardiac organoid treated in parallel but in the absence of the test compound.

The processes and the cardiac organoid have the advantage of being available on the basis of cultivated pluripotent stem cells, and have the advantage of providing adjoining layers comprising or consisting of different cardiac cells, optionally including non-cardiac cells, which layers are grown by a purely in vitro process, and to provide an in vitro model of adjoining cardiac tissue layers, optionally including non-cardiac cells. These tissue layers have been found to be well-structured and to contain cell types, which layers and cell types are typical of the early embryonic heart. The cardiac organoids can be kept in cell culture medium in static or agitated, e.g. shaken, culture vessels, e.g. without directional fluid flow, without blood flow, and freely suspended, i.e. without mechanical fixation.

### State of the art

It is known to cultivate cardiac cells in order to obtain layers of these cells which adhere to a cultivation vessel surface.

The trademark Matrigel (Corning Life Sciences and BD Bioscience) is known to designate a gelatinous protein mixture secreted by Engelbreth-Holm-Swarm (EHS) mouse sarcoma cells.

Richards et al., Biomaterials 112-123 (2017) describe the fabrication of cardiac organoids by depositing cardiomyocytes, cardiac ventricular fibroblast cells, umbilical vein endothelial cells (HUVEC), human adipose-derived microvascular endothelial cells (HAMEC), and human adipose-derived stem cells (hADSC) in agarose moulds to first generate spherical microtissues which are then assembled to spheroids.

Elliott et al. (2011) "NKX2-5(eGFP/w) hESCs for isolation of human cardiac progenitors and cardiomyocytes", Nature methods 8 (12), S. 1037-1040. DOI: 10.1038/nmeth.1740 describe the generation of hESC3 NKX2.5-eGFP cells.

### Object of the invention

It is an object of the invention to provide an alternative process for producing cardiac tissue in an in vitro process, which cardiac tissue shall form at least two layers comprising or consisting of different cells, which can be cardiac cells, optionally in combination with other cell types.

### Description of the invention

The invention achieves the object by the features of the claims, especially by an in vitro process for producing a cardiac organoid, the process comprising or consisting of the following order of steps of
a) providing cultivated pluripotent stem cells (PSC), preferably only PSC, wherein PSC preferably are human PSC, e.g. in a cell number of at least 50 or at least 100 or at least 1000 cells, e.g. up to 200,000 or up to 100,000 cells, e.g. 4,000 to 10,000 cells, preferably 5000 cells, in a suspension in a first culture medium, in a first vessel,
b) centrifuging the PSC in the first vessel having a U-shaped bottom to localize the PSC at the bottom of the first vessel,
c) incubating the PSC localized at the bottom of the first vessel under the first medium under cell culture conditions to form one aggregate of PSC,
d) optionally removing the first medium from the aggregate of PCS localized at the bottom of the first vessel,
e) embedding the aggregate of PCS in hydrogel, preferably by positioning a volume of hydrogel in a second vessel and transferring the aggregate of PCS localized at the bottom of the first vessel into the volume of hydrogel,
f) incubating the aggregate of PCS embedded within the hydrogel for solidifying the hydrogel, e.g. for at least 10 min, e.g. für 10 to 90 min or to 45 min, or for 45 to 90 min
g) covering the aggregate of cells embedded in the solidified hydrogel with a second cell culture medium and incubating under cell culture conditions, preferably for 1 to 3 d, e.g. for 36 to 60 h,
h) removing the second medium from the cells and adding a third cell culture medium containing a first differentiation factor having activity to induce the WNT pathway and incubating under cell culture conditions for at least 6 h, e.g. for up to 3 d, preferably for 12 to 48 h, e.g. for 24h,
i) removing the third medium from the cells and adding a fourth cell culture medium not containing the first differentiation factor or containing an agent neutralizing the activity of the first differentiation factor, and incubating under cell culture conditions for at least 6 h, e.g. up to 3 d, preferably for 12 to 48 h, e.g. for 24 h,
j) removing the medium from the cells and adding a fifth cell culture medium containing a second differentiation factor having activity to inhibit the WNT pathway and incubating under cell culture conditions for at least 1 d or at least 2 d, preferably for 46 to 50 h, e.g. for 48 h,
k) removing the fifth medium from the cells and adding a sixth medium not containing insulin and not containing a differentiation factor having activity to induce or to inhibit the WNT pathway and incubating under cell culture conditions for at least 1 d or at least 2 d, preferably for 2 days,
l) removing the sixth medium from the cells and adding a seventh cell culture medium containing insulin and incubating under cell culture conditions for at least 1 d or at least 2 d, preferably for 2 days,
m) and subsequently change the seventh medium for fresh cell culture medium at least every 2 d.

As an alternative, PSC can be replaced by omnipotent or totipotent stem cells. Accordingly, in the process the stem cells can optionally be at least pluripotent stem cells.

Cultivated pluripotent stem cells (PSC) preferably are characterized by presence of the markers Tra-1-60 and SSEA4.

The first vessel preferably has a low-attachment surface, preferably an ultra-low attachment surface which prevents attachment of the PSC to the vessel. The first vessel can e.g. be contained in a microtiter plate, e.g. in a 96-well plate. The second vessel preferably has a low-attachment surface, preferably an ultra-low attachment surface which prevents attachment of the PSC to the vessel. The second vessel may have a flat or U-shaped bottom. The first vessel and the second vessel can have the same shape and/or surface properties.

Generally, the U-shaped bottom of the first and second culture vessels can be a tapering concave bottom, e.g. round or stepped or having angled surfaces, e.g. having a concave pyramid shape or V-shape.

The first medium preferably is E8 medium, having the composition of DMEM/F12, L-ascorbic acid-2-phosphate magnesium (64 mg/L), sodium selenium (14 µg/L), FGF2 (100 µg/L), insulin (19.4 mg/L), NaHCO₃ (543 mg/L) and transferrin (10.7 mg/L), TGFβ1 (2 µg/L) or NODAL (100 µg/L). Osmolarity of all media was adjusted to 340 mOsm at pH7.4, in order to provide for pluripotency of the ESC. Preferably, the first medium is supplemented with ROCK inhibitor, e.g. 10 µM ROCK inhibitor (Rho-associated-kinase inhibitor).

The volume of each medium can be in the range of 100 µL to 300 µL, especially 150 µL to 250 µL for every 5000 cells.

The centrifugation in step b), e.g. twice at 300 x g in a centrifuge temperature-controlled to 4°C, serves to localize the PSC on the bottom of the first vessel, e.g. to collect the PSC. Following the centrifugation, the first vessel is placed in an incubator essentially without moving the first medium in relation to the first vessel, in order to maintain the PSC localized close together. The first medium remains in the first vessel, also in step c).

The incubation of the PSC localized on the bottom of the first vessel under cell culture conditions and without agitation in step c). This incubation, e.g. for 24h, results in the PSC to form one aggregate of cells.

Removing the first medium from the aggregate of cells in step d) serves to minimize or avoid the transfer of first medium into the hydrogel that is positioned in the U-shaped bottom of a second vessel in step e), when transferring the aggregate of cells into the hydrogel in step f). Transferring the aggregate of cells in step e) can e.g. be done using a pipette having a wide opening in order to avoid shearing the aggregate of cells.

The hydrogel is for cell culture, e.g. is permeable for dissolved oxygen and permeable for medium components and preferably permeable for metabolic products, e.g. permeable for diffusion. Preferably, the hydrogel is on the basis of an extracellular matrix of basal membranes of animal cells. The hydrogel can be on the basis of laminin and/or entactin and/or collagen and/or fibronectin and/or PEG, optionally on the basis of PEG (polyethylene glycol), e.g. in combination with fibronectin. The hydrogel preferably has a gelatinous structure. A preferred hydrogel is Matrigel.

The hydrogel, e.g. Matrigel, preferably has a protein concentration of 9.7 to 10.1 mg/mL. In step e), preferably a volume of 15 to 30 µL, preferably 20 µL Matrigel is positioned onto the U-shaped bottom of a second vessel and the aggregate of PCS is positioned into the Matrigel for embedding the aggregate of PCS within the Matrigel. The second vessel preferably has a low-attachment surface, preferably an ultra-low -attachment surface. The second vessel can have the same specifications as the first vessel.

The incubation of step f), e.g. for 45 to 60 min under cell culture conditions, serves to solidify the hydrogel prior to adding the second culture medium in step g).

In step g), the second medium can have the same composition as the first medium, e.g. medium E8 . The volume of the second medium in step g) can be 80 to 150 µL, e.g. 100µL. The incubation of step g) is for at least 1 day or at least two days, preferably up to 3 days, preferably for 36 to 60 h.

Removal of the second medium and adding the third medium containing a first differentiation factor in step i) serves to activate the WNT pathway. The first differentiation factor having activity to induce the WNT pathway preferably is an inhibitor of GSK3beta (glycogen synthase kinase 3 beta), and preferably has no effect or cross-reactivity on CDKs (cyclin-dependent kinases). A preferred first differentiation factor is CHIR99021 (CHIR, 6-[[2-[[4-(2,4-dichlorophenyl)-5-(5-methyl-1H-imidazol-2-yl)-2 pyrimidinyl]amino]ethyl]amino]-3-pyridinecarbonitrile), e.g. at 7.5µM, e.g. using 200µL of the third medium per second vessel. The third medium preferably is RPMImedium containing B27 supplement without insulin and it additionally contains the first differentiation factor.

Preferably, after step h), the third medium containing the first differentiation factor is removed in step i), preferably at 24h after adding this third medium in step j), and a fourth cell culture medium is added, which does not contain a differentiation factor, e.g. no first differentiation factor, and incubating under cell culture conditions, e.g. for 1d. When the process includes step i), this is presently considered to reduce the WNT activation induced the first differentiation factor. The fourth medium preferably is free from insulin, e.g. RPMI medium containing B27 supplement but without insulin.

In step j), the medium is removed from the cell aggregate and a fifth cell culture medium is added, which contains a second differentiation factor which inhibits the WNT2 pathway, and preferably the fifth medium contains no insulin. The fifth medium can be RPMI medium containing B27 supplement but without insulin. The second differentiation factor preferably is an inhibitor of the WNT pathway activator Porcupine, e.g. IWP2 (Inhibitor of WNT Production-2, CAS No. 686770-61-6), e.g. at a concentration of 5µM, e.g. using 100µL to 300 µL, e.g. up to 200 µL medium, per second vessel.

In step 1), preferably after 2 d incubation of step k), the fifth medium is removed and a sixth medium is added to the agglomerate, which medium does not contain a first nor a second inhibitor. The sixth medium preferably contains no insulin. The sixth medium can e.g. be RPMI medium containing B27 supplement without insulin. In step k), the volume of the sixth medium can e.g. be 100 µL to 300 µL, e.g. 150 to 250 µL, preferably 200 µL per second vessel. RPMI medium is RPMI1640 (Inorganic Salts: Calcium nitrate * 4H₂O (0.1 g/L), magnesium sulfate (0.04884 g/L), potassium chloride (0.4 g/L), sodium bicarbonate (2 g/L), sodium chloride (6 g/L), sodium phosphate dibasic (0.8 g/L); Amino Acids: L-alanyl-L-glutamine (0 g/L), L-arginine (0.2 g/L), L-asparagine (0.05 g/L), L-aspartic acid (0.02 g/L), L-cystine * 2HCl (0.0652 g/L), L-glutamic acid (0.02 g/L), glycine (0.01 g/L), L-histidine (0.015 g/L), hydroxy-L-proline (0.02 g/L), L-isoleucine (0.05 g/L), L-leucine (0.05 g/L), L-lysine * HCI (0.04), L-methionine (0.015 g/L), L-phenylalanine (0.015 g/L), L-proline (0.02 g/L), L-serine (0.03 g/L), L-threonine (0.02 g/L), L-tryptophan (0.005 g/L), L-tyrosine * 2Na * 2H₂O (0.02883 g/L), L-valine (0.02 g/L); Vitamins: D-biotin (0.0002 g/L), choline chloride (0.003 g/L), folic acid (0.001 g/L), myo-inositol (0.035 g/L), niacinamide (0.001 g/L), p-aminobenzoic acid (0.001 g/L), D-phantothenic acid (hemicalcium) (0.00025 g/L), pyridoxine * HCl (0.001 g/L), riboflavin (0.0002 g/L), thiamine * HCl (0.001 g/L), vitamin B12 (0.000005 g/L);
D-glucose (2 g/L), glutathione (0.001 g/L), phenol red * Na (0.0053 g/L); L-Glutamine (0.3 g/L), sodium bicarbonate (0 g/L)).

In step 1), the sixth medium is removed and replaced by a seventh medium which contains insulin, e.g. RPMI medium containing B27 supplement with insulin added.

In step m) the seventh medium of step 1) is replaced by fresh cell culture medium, preferably fresh seventh medium, which preferably contains added insulin.

Preferably, the incubation in steps c) to m), preferably in steps c) to h), are under static conditions, e.g. without shaking or moving of the vessel. Optionally, the incubation starting from step m) are with mild agitation, e.g. under orbital shaking, preferably in a larger volume of medium and in a vessel for cell culture with orbital shaking.

The process for producing the cardiac organoids has the advantage that only one type of cells, namely PSC, can be used in the process and that during the process the PSC differentiate and self-organize into a three-dimensional structure which comprises adjoining layers comprising or consisting of different cardiac cell types, e.g. without mechanically manipulating cells into a specific layered structure, and without initially providing different cardiac cell types.

Herein, the gelatinous protein mixture secreted by Engelbreth-Holm-Swarm (EHS) mouse sarcoma cells is generally referred to as Matrigel.

Generally, all media can contain anti-bacterial agents, e.g. penicillin and/or streptomycin for the prevention of bacterial contaminations.

For analysing the effect of a test compound, the test compound can be added in any of the steps of the process. For analysing the effect of a test compound, the test compound can be added to the medium in at least one of steps e) to m, e.g. in at least one of steps i) to m), preferably after a total of 10 to 13 days after step i).

The process for producing the cardiac organoids has the advantage of reproducibly generating cardiac organoids of one structure, e.g. having an outer diameter of 0.5 to 5 mm or up to 3 or up to 2.5 mm, e.g. of 1.2 to 3 mm or up to 2.5 mm. The structure of the cardiac organoids contains or consists of a first layer, comprising or essentially or consisting of endothelial cells, which first forms an inner part and has cavities, which preferably comprise hemogenic endothelium and/or SOX17-positive cells, a second layer directly adjacent the first layer and surrounding only a portion of the first layer, the second layer forming dense myocardium, optionally also endocardium, comprising or essentially consisting of NKX2.5-positive cardiomyocytes, and opposite the first layer a third layer directly adjacent to the second layer only and surrounding only a portion of the second layer, which third layer forms epicardium and comprises or essentially consists of epicardial cells and cardiomyocytes, has blood vessel-like structures and forms a loose tissue from epicardial cells and cardiomyocytes, wherein the third layer can be covered by a directly adjacent fibroblast layer. Therein, preferably the first layer completely surrounds the cavity, i.e. the first layer is circumferentially closed, and the second layer surrounds the first layer only partially, leaving a portion of the first layer non-covered from another layer. The third layer surrounds the second layer only partially, e.g. the third layer covers the second layer up to a ring-shaped section of the second layer, which ring-shaped section of the second layer is non-covered, which ring-shaped section is adjacent to the non-covered portion of the first layer.

The structure of the cardiac organoids therefore comprises or consists of, e.g. in one perspective, at least one non-covered portion of the first layer surrounded by a ring-shaped section of the second layer, wherein the second layer is covered by the adjacent third layer opposite the first layer, and the ring-shaped section of the second layer is not covered by the third layer. The structure of the cardiac organoids preferably has at least one surface area which is formed by a non-covered portion of the first layer, by a ring-shaped section of the second layer directly adjacent the first non-covered portion of the first layer, and directly adjacent to the ring-shaped section of the second layer, the third layer which covers and surrounds the second layer, the third layer bordering on the ring-shaped section of the second layer, and preferably a fourth layer, e.g. of loose cardiac fibroblasts, only adjacent the third layer. Optionally, the first layer in its part directly under its non-covered portion contains less or no cavities than its portions which are covered by the second layer. In the structure of the cardiac organoids, the at least one surface area can be formed by the ring-shaped section of the second layer, which encloses the non-covered portion of the first layer and, opposite the non-covered portion of the first layer, by the third layer surrounding the ring-shaped section of the second layer, wherein the third layer preferably covers the second layer completely and borders on the ring-shaped section of the second layer.

As the process for producing the cardiac organoids reproducibly generates the cardiac organoids having this structure, the effect of a test compound added during the process or to cardiac organoids having a pre-determined size, can be analysed, e.g. by determining structural differences to cardiac organoids produced without the addition of the test compound.

The structure of the cardiac organoids has the advantage of allowing direct observation of all the layers from the perspective directed onto the non-covered portion of the first layer and/or onto the ring-shaped section of the second layer.

Optionally, a section of the cardiac organoids, e.g. at least one of its layers, can be for use as a tissue implant for implantation into a patient, e.g. as a tissue implant, e.g. as a replacement or filler for a defect tissue.

Optionally, the pluripotent stem cells originate from a patient, e.g. from a tissue sample taken from a patient. The patient can be diagnosed to have a disease or can be suspected of having a disease, and the process for producing the cardiac organoids can be used to analyse the effect of a test compound onto the cardiac organoids, e.g. during or following their development to a pre-determined size, wherein the test compound can be a pharmaceutical compound for use in the treatment of the disease. Such a process can be used to analyse the effect of the pharmaceutical compound prior to administration of the compound to the patient. Alternatively or additionally, cardiac organoids produced according to the process of the invention from pluripotent stem cells that originate from a patient can be used to produce a cardiac organoid for use of at least one of its layers as a replacement for a defect tissue in the patient.

The PSC preferably are a cell line, e.g. HES3.

The invention is now described in greater detail by way of examples with reference to the figures, which show in
- Fig. 1 a schematic representation of a preferred embodiment of the process,
- Fig. 2 FACS results for an exemplary PSC cell line,
- Fig. 3 fluorescence microscopic picture of a cardiac organoid obtained on day 14 of the process (d10),
- Fig. 4 fluorescence microscopic pictures of different cardiac organoids on day 14 of the process,
- Fig. 5 shows a fluorescence microscopic picture of a cardiac organoid using immunostaining of Calponin-positive myofibroblasts at the perimeter of the outer layer of cardiac organoids,
- Fig. 6 shows expression of WT1 in the outer layer only of cardiac organoids, 6A in immuno-staining, and 6B expression of a reporter gene,
- Fig. 7 shows a fluorescence microscopic picture of a cardiac organoid using immunostaining of cTnT in a paraffin section of a cardiac organoid,
- Fig. 8 shows cardiac organoids with staining for endothelial cells, in 8A a fluorescent micrograph for NKX2.5-eGFP expression with DAPI staining, in 8B a fluorescent micrograph with staining for CD31 and DAPI staining, in 8C a fluorescent micrograph with NKX2.5-eGFP expression, staining for CD31 and with DAPI staining,
- Fig. 9 shows a micrograph of a cardiac organoid with immunostaining for SOX17,
- Fig. 10 shows a micrograph of a paraffin section of a cardiac organoid with DAPI staining,
- Fig. 11 shows a micrograph of a paraffin section of a cardiac organoid stained for vascular endothelial cadherin,
- Fig. 12A and 12B show light micrographs and Fig. 12C an electron micrograph in a section of a cardiac organoid in the region of a large cavity,
- Fig. 13 shows an electron micrograph of a section of a large cavity of a cardiac organoid,
- Fig. 14 shows a micrograph of a cardiac organoid after long-term culture stained with Dil-Ac-LDL,
- Fig. 15 shows a schematic representation of the structure of cardiac organoids produced by the process of the invention,
- Fig. 16 shows micrographs of cardiac organoids treated with Thalidomide and untreated cardiac organoids, and
- Fig. 17 graphically shows quantitative differences in the structure of cardiac organoids treated with Thalidomide and without test compound added.

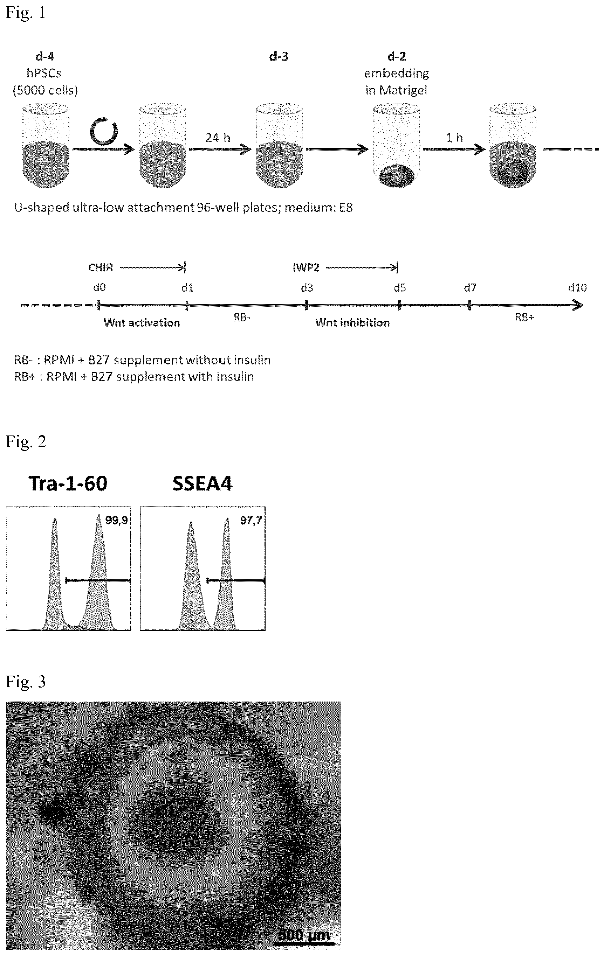

Fig. 1 shows a schematic overview of the process, wherein the sequence of steps is counted from the start of step h) as day 0. Therein, step a) is performed at day -4 (d-4) by centrifuging approx. 5000 human PSC (hPSCs) in the wells of a 96-well microtiter ultra-low attachment plate with U-shaped bottom as the first vessel to generate the aggregate of PSC at the U-shaped bottom of step b). The aggregate of PSC are incubated in the medium in step c) for the preferred duration of 24 h, ending on day -3 (d-3). The result of steps d) and e), which is the cell aggregate obtained on d-3 embedded in hydrogel in the bottom of a second vessel, is shown on day -2 (d-2) as the result of step e). The cell aggregate embedded in hydrogel is incubated for 1 h under cell culture conditions in step f), without medium contained in the second vessel. In step g) the second medium is added and incubation is for 2 d until on day 0 (d0) in step h) the second medium is replaced by the third medium containing the first differentiation factor to start the WNT pathway, with incubation in the third medium for 1 day (until day 1 (d1)). The third medium was 200 µL with 7.5 µM CHIR99021 per second vessel.

The step i) of replacing the third medium for a fourth medium that is free of a differentiation factor, is made on day 1 and incubation in the fourth medium is for 2 days until day 3 (d3). In the alternative, step i) can be performed with an incubation of 1 to 2 days, preferably 24 h under cell culture conditions, and the third medium is subsequently directly removed and exchanged for the fifth medium containing an inhibitor of the WNT pathway, IWP2 at 5µM, 200 µL medium per second vessel in step j). Incubation was for 2 days until day 5 (d5), when in step k) the fifth medium was removed and replaced by a sixth medium which is free from a differentiation factor and preferably contains no insulin, and incubation is for 2 days until day 7 (d7). The sixth medium is replaced by a seventh medium, which is generally free from differentiation factors and preferably contains insulin, e.g. B27 supplement, followed by incubation for at least 1 day, e.g. for up to 3 days, until day 10 (d10) in step 1). Following step 1), the sixth medium is exchanged for fresh medium, preferably containing insulin, in order to maintain viability, e.g. every day to every 2 days in step m).

In Fig. 1, RB- is RPMI medium containing B27 supplement without insulin, RB+ is RPMI medium containing B27 supplement, which contains insulin. B27 supplement, e.g. available from ThermoFisher Scientific, contains biotin, DL-alpha-tocopherol acetate, DL-alpha-tocopherol, vitamin A (acetat), BSA, fatty acid free Fraction V, catalase, human recombinant insulin, human transferrin, superoxide dismutase, corticosterone, D-galactose, ethanolamine HCl, glutathione (reduced), L-carnitine HCl, linoleic acid, linolenic acid, progesterone, putrescine 2HCl, sodium selenite, T3 (triodo-1-thyronine). An insulin-free variant of B27 supplement is commercially available.

### Example 1: In vitro production of cardiac organoids only from PSC

The process was performed for steps a) to n) as described with reference to Fig. 1. Incubation under cell culture conditions was in a 5% CO₂ atmosphere at 37°C, using ultra-low attachment 96 well microtiter plates, Nunclon Sphera 96U-well plate available from Thermo Fisher Scientific, as the first vessels. The PSC were the human embryonic stem cell line hESC3 NKX2.5-eGFP. These exemplary PSC were genetically manipulated to express enhanced green fluorescent protein (eGFP) as a reporter under the control of the early cardiomyocyte-specific marker NKX2.5 to indicate early cardiomyocyte differentiation.

These PSC were cultivated on cell culture flasks (preferably Geltrex-coated) in E8 medium and detached from the flasks to provide the PSC in suspension. In step a), 5000 PSC suspended in 100 µL of the first medium, which was E8 medium (DMEM/F12, L-ascorbic acid-2-phosphate magnesium (64 mg/L), sodium selenium (14 µg/L), FGF2 (100 µg/L), insulin (19.4 mg/L), NaHCO₃ (543 mg/L) and transferrin (10.7 mg/L), TGFβ1(2 µg/L) or NODAL (100 µg/L), osmolarity adjusted to 340 mOsm at pH7.4) supplemented with 10µM ROCK inhibitor (Y-27632, catalogue No. 72302, Stemcell Technologies)), were carefully dispensed into wells of the microtiter plate. Centrifugation of step b) was twice for 3 min at 300 x g with the centrifuge cooled to 4°C. The centrifuged first vessels each contained one agglomerate on the U-shaped bottom and were carefully placed into a cell culture incubator in order to avoid disturbance of the cells localized at the U-shaped bottom. Incubation of step c) without agitation was for 24 h, resulting in the formation of an aggregate of the cells. For embedding in Matrigel (thawed on ice, 9.7 to 10.1 mg/mL protein content) as the exemplary hydrogel, 20 µL Matrigel were positioned in each well of a microtiter plate, the wells of which forming second vessels, which plate was of the same ultra-low attachment quality as the first. As preferred, the medium was removed from the cell aggregates contained in the first vessels, and the cell aggregates were singly and carefully transferred into the Matrigel droplets contained in the second vessels. It was found that for the transfer of a cell aggregate, a pipette set to approx. 3µL and equipped with a cut tip having a large opening could be used. The second vessel, i.e. the second microtiter plate, was re-positioned into the incubator for 45 to 60 min, which allowed for a solidification of the Matrigel. Then, 100 µL E8 medium but without ROCK inhibitor was dispensed into each second vessel and the second vessel was incubated for 2 days without agitation according to step h). After the incubation of step h), on day 0, differentiation was started by completely removing the E8 medium and adding 200 µL RPMI medium supplemented with B27 supplement without insulin and containing 7.5 µM CHIR99021 into each second vessel, and incubated for exactly 24h according to step h). Then, this third medium was exchanged for a fourth medium consisting of RPMI medium with B27 supplement without insulin, and incubation was for 2d. Then, the fourth medium was removed and exchanged for 200 µL RPMI medium with B27 supplement without insulin and containing 5µM IWP2 as the second differentiation factor in each second vessel according to step k), with incubation for 48 h. Subsequently, the medium was exchanged every 2 days for RPMI medium with B27 supplement containing insulin.

Fig. 2 shows the result of a FACS analysis of the suspended PSC cells with staining for the pluripotency markers Tra-1-60 (99.9% of cells positive) and SSEA4 (97.7% of cells positive), confirming pluripotency of the cells at the beginning of the process.

Fig. 3 shows a fluorescence microscopic picture of a typical cardiac organoid generated from the reporter hESC line HES3 NKX2.5^{eGFP}. The cardiac organoid shows an inner part forming an inner layer 1, which is comprised of NKX2.5-eGFP-negative cells. The inner layer is surrounded by a dense ring of NKX2.5-eGFP-positive cardiomyocytes, which can also be described as a myocardial ring. The dense ring is surrounded by a ring-like structure of an outer layer, which contains both NKX2.5-eGFP-positive and NKX2.5-eGFP-negative cells. The outer layer seems to be loose tissue.

It was generally observed that the cardiac organoids start to regularly contract after approx. 11 days of the process (day 7).

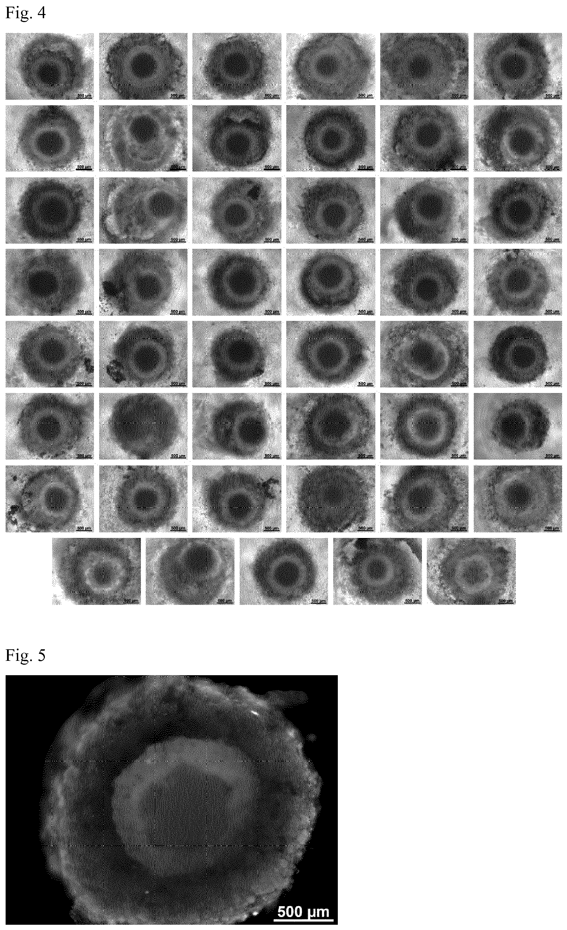

Fig. 4 depicts fluorescence microscopic pictures of different cardiac organoids on day 14 (d10) of the process, showing very similar structures and NKX2.5-eGFP expression for the organoids produced in parallel in one batch. This results shows that the process reproducibly generates the cardiac organoids having a similar structure.

Fig. 5 depicts a cardiac organoid in a whole-mount immunofluorescence stain using a Cy5-labelled anti-Calponin antibody (Calponin 1 antibody (CALP): sc-58707 von Santa Cruz Biotechnology), DAPI staining and fluorescence of NKX2.5-eGFP. This result shows that the cardiac organoids of the invention contain Calponin-positive myofibroblasts at the perimeter of the outer layer.

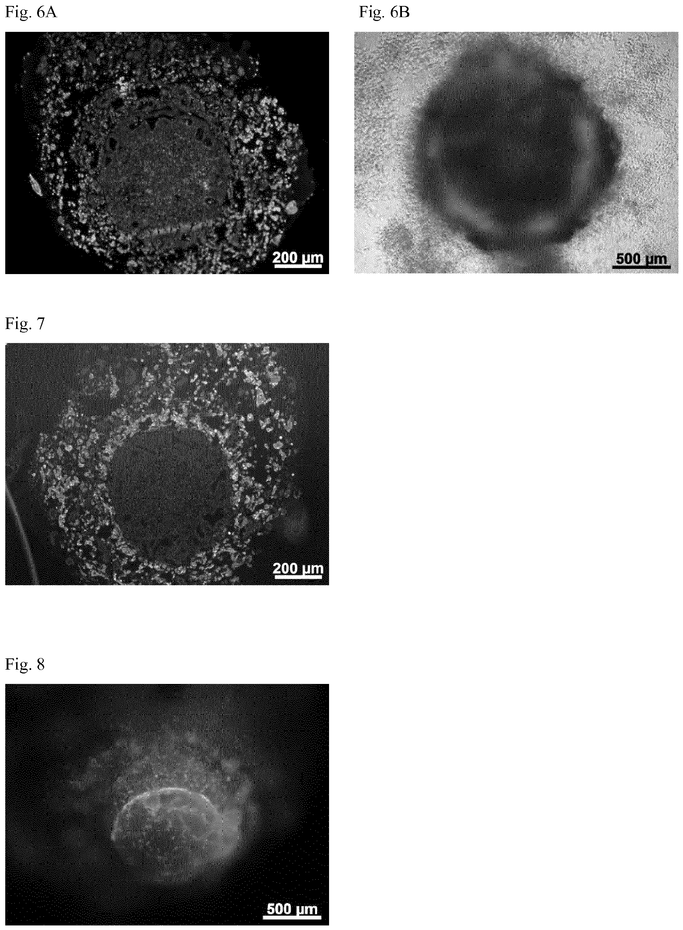

Fig. 6 depicts micrographs of a cardiac organoid with WT1 immunostaining using an Alexa488-labelled anti-WTl antibody with DAPI staining in a paraffin section in 6A, and in 6B an immunofluorescence micrograph of a cardiac organoid expressing eGFP under the control of the WT1 promoter (HES3 WT1^{eGFP} cells). These results show that epicardial cells in the cardiac organoids are exclusively found in the out layer and not in other parts of the organoids.

Fig. 7 shows a paraffin section of a cardiac organoid with immuno-staining of the myocardial marker troponin T (cTnT) using a Cy3-labelled anti-cTnT antibody (Cardiac Troponin T Antibody (13-11) obtained from Invitrogen), with DAPI staining. This result shows that the cardiac organoids contain a dense ring of cTnT-positive cells between the outer layer and the inner part. This dense ring can consist of cTnT-positive cells, which are myocardial cells. Further, cTnT-positive cells are found in the outer layer.

Fig. 8 shows a whole-mount fluorescence micrograph of a cardiac organoid expressing NKX2.5-eGFP with DAPI staining and immunofluorescence staining for CD31 using a Cy3-labelled anti-CD31 antibody (CD31, Endothelial Cell (Concentrate) Clone JC70A obtained from Agilent, Dako). This shows that the cardiac organoids contain endothelial cells in their inner part.

Further, Figure 8 shows a line between the myocardial ring and the inner part, which could show a dense layer of endothelial cells, and which could indicate endocardium.

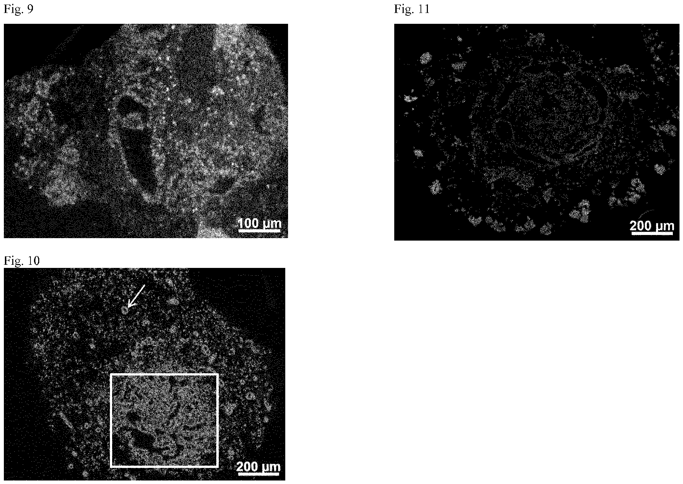

Fig. 9 shows a micrograph of a cardiac organoid in a section with immunostaining for SOX17 using a labelled anti-SOX17 antibody (Human SOX17 Antibody (AF1924) obtained from R&D Systems). This section shows that endothelial cells of the endothelial network inside the cardiac organoids are SOX17-positive. Noticeably, SOX17-positive endothelial cells are found in the aorta-gonad-mesonephros region of the developing embryo.
Fig. 10 depicts a micrograph of a paraffin section of a cardiac organoid with DAPI staining. This shows that the organoids contain large cavities in the center of their inner part, highlighted by the drawn box, and small, vessel-like structures in the outer loose tissue, indicated by the arrow.

The micrograph of Fig. 11 using immuno-staining of the endothelial marker vascular endothelial cadherin (VE-cadherin or CD144) with a labelled anti-CD 144 antibody human CD144 antibody BMS158 obtained from eBioscience) shows that the blood vessel-like structures of the outer part of the cardiac organoids contain or consist of CD144-positive cells.

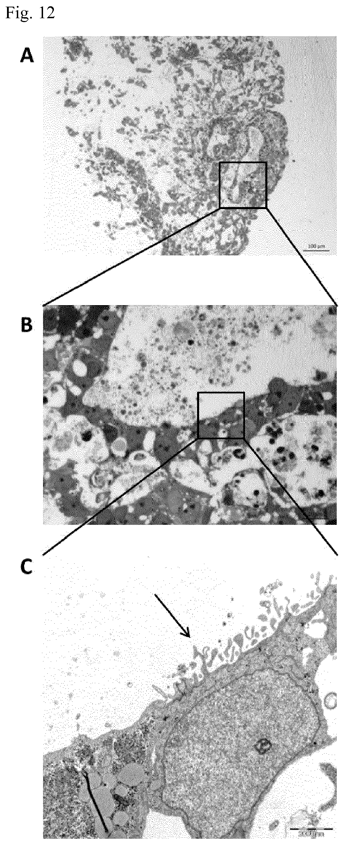

Fig. 12A and the enlargement in Fig. 12B show semi-thin sections of cardiac organoids embedded in epoxy resin, in staining with toluidine blue in light microscopy, and in Fig. 12C enlarged by electron microscopy. These pictures show that the large cavities of the cardiac organoids are lined with epithelium having pseudopodia (indicated by the arrow in Fig. 12C).

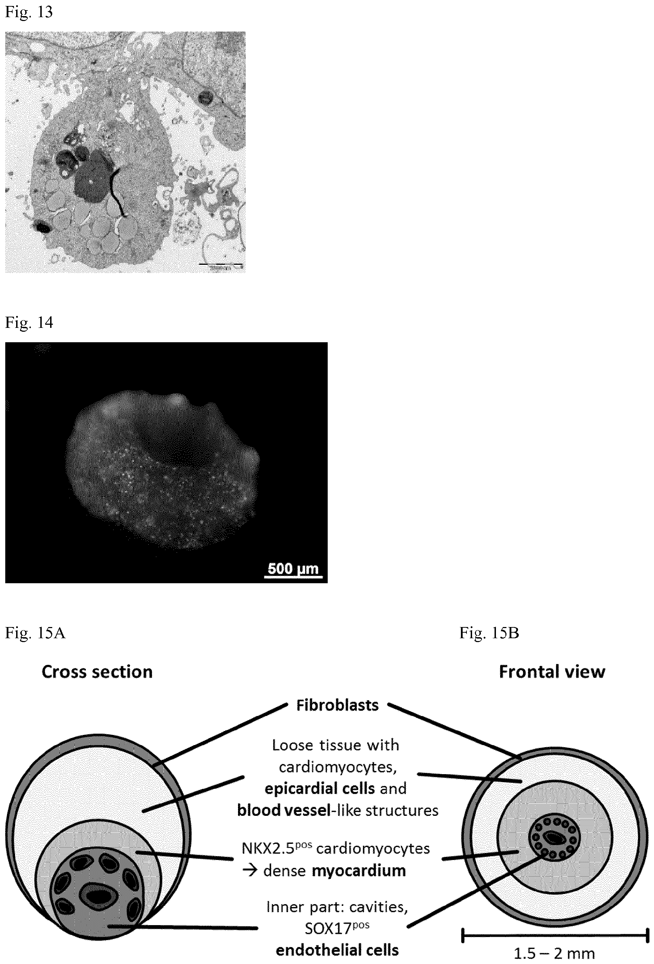

The electron micrograph of Fig. 13 shows a cell that was found within a large cavity of a cardiac organoid. This cell has a morphology suggesting a macrophage phenotype. This finding supports that the inner part of the cardiac organoids contains hemogenic endothelium.

### Example 2: Long-term culture of cardiac organoid

After 14 days of the process, i.e. on day 10, cardiac organoids produced according to Example 1 were transferred to wells of a 12-well plate containing PBS (phosphate-buffered saline, pH 7.4), the PBS was removed by suction and the Matrigel was dissolved by adding Cell Recovery Solution, obtained from Corning, according to the manufacturer's instructions. Then, the organoid was gently washed with PBS, PBS was removed, and 2 mL RPMI medium with B27 supplement and containing insulin was added into each well, then the plate was incubated further, with exchange of the medium for fresh medium every 3 to 4 days. The organoid shown in Fig. 14 was kept in this culture until day 146 (i.e. 146 days starting from step i), then stained with Dil-AC-LDL (obtained from Thermo Fisher Scientific). Staining procedure: Dil-Ac-LDL was added directly to the medium (RB+) to a final concentration of 5 µg/mL. Organoids were stained in a 12-well plate in a volume of 2 mL. Incubation with the DiI-Ac-LDL: 4 h in the incubator. Afterwards, the medium with the DiI-Ac-LDL was removed and fresh medium (RB+) without the dye was added. For imaging, medium was replaced by PBS. The fluorescence micrograph of Fig. 14 shows that the organoid still contains endothelial cells and expresses NKX2.5-eGFP. The cardiac organoid showed contractions until the end of the culture.

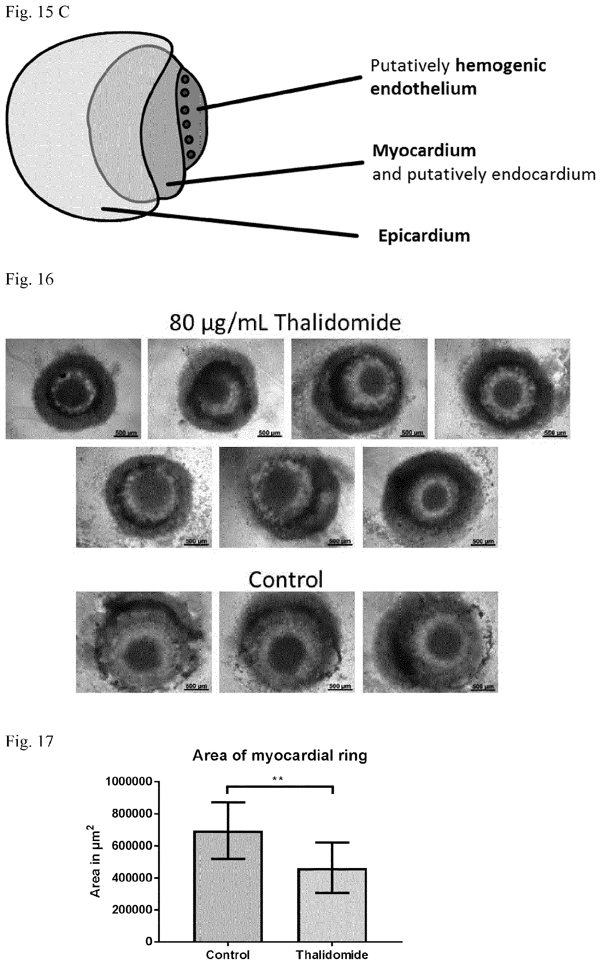

Figures 15A-15C show a schematic representation of the structure of the cardiac organoids which could be derived from the analytical results. The cardiac organoids produced by the process of the invention contain or consist of a first layer, comprising or essentially consisting of endothelial cells, which first forms an inner part and has cavities, which preferably comprise hemogenic endothelium and/or SOX17-positive cells, a second layer directly adjacent the first layer and surrounding only a portion of the first layer, the second layer forming dense myocardium, optionally also endocardium, comprising or essentially consisting of NKX2.5-positive cardiomyocytes, and a third layer directly adjacent to the second layer only and surrounding only a portion of the second layer, which third layer forms epicardium and comprises or consists of epicardial cells and cardiomyocytes, has blood vessel-like structures and forms a loose tissue from epicardial cells and cardiomyocytes, wherein the third layer is covered by a directly adjacent fibroblast layer.

### Example 3: Testing of the effect of a test compound on cardiac organoids

As an exemplary test compound, Thalidomide was used to test its influence on the development of cardiac organoids during the production process. Cardiac organoids were produced according to Example 1, and on day 1 (d1), i.e. after the incubation for 1 day in step i), 80 µg/mL Thalidomide was added to the culture medium. Micrographs of 7 cardiac organoids treated in parallel with Thalidomide (80 µg/mL Thalidomide) are shown in Fig. 16 along with micrographs of three cardiac organoids that were produced in parallel but without addition (Control) of the test compound.

A comparison of the micrographs of the Thalidomide-treated cardiac organoids to the Control makes it clear that Thalidomide results in the absence of any NKX2.5-eGFP-positive cells on the outer layer, and that the myocardial ring of the Thalidomide-treated cardiac organoids is substantially thinner than in Controls. The average diameter of the myocardial ring in Controls was 276 µm, with Thalidomide it was 165 µm, which result is also shown in Fig. 17.

This result on the exemplary test compound shows that the known effect of Thalidomide to cause also cardiac malformations during embryo development is also seen in the development of the cardiac organoids in the process according to the invention. This shows that the process for producing cardiac organoids is a suitable in vitro assay for the effect of test compounds, e.g. in respect of teratogenic effects.

## Claims

1. Process for in vitro producing a cardiac organoid comprising
a) providing cultivated pluripotent stem cells (PSC) in a suspension in a first culture medium,
b) centrifuging the PSC in a first vessel having a U-shaped bottom to localize the PSC at the bottom of the first vessel,
c) incubating the PSC localized at the bottom of the first vessel under the first medium under cell culture conditions,
d) optionally removing the first medium from the PCS localized at the bottom of the first vessel,
e) embedding the PCS within hydrogel,
f) incubating the PCS embedded within the hydrogel for solidifying the hydrogel,
g) covering the cells embedded in the solidified hydrogel with a second cell culture medium and incubating under cell culture conditions,
h) removing the second medium from the cells and adding a third cell culture medium containing a first differentiation factor having activity to induce the WNT pathway and incubating under cell culture conditions for at least 6 h,
i) removing the third medium from the cells and adding a fourth cell culture medium not containing a differentiation factor or containing an agent neutralizing the activity of the first differentiation factor, and incubating under cell culture conditions for at least 6 h,
j) removing the medium from the cells and adding a fifth cell culture medium containing a second differentiation factor having activity to inhibit the WNT pathway and incubating under cell culture conditions for at least 1 d,
k) removing the fifth medium from the cells and adding a sixth medium not containing insulin and not containing a differentiation factor having activity to induce or to inhibit the WNT pathway, and incubating under cell culture conditions for at least 1 d,
l) removing the sixth medium from the cells and adding a seventh cell culture medium containing insulin and incubating under cell culture conditions for at least 1 d,
m) and subsequently change the seventh medium for fresh cell culture medium at least every 2 d.

2. Process according to claim 1, **characterized in that** the incubation in steps a) to m) is under essentially static conditions.

3. Process according to one of the preceding claims, **characterized in that** in step c) the PSC localized at the bottom of the first vessel under the first medium under cell culture conditions are incubated for 46 to 50 h.

4. Process according to one of the preceding claims, **characterized in that** in step e) hydrogel is positioned in a second vessel and the aggregated PCS are transferred from the first vessel into the hydrogel for embedding the aggregate of PCS within the hydrogel.

5. Process according to one of the preceding claims, **characterized in that** in step g) the PCS embedded in the solidified hydrogel are incubated with a second cell culture medium for at approximately 2 d.

6. Process according to one of the preceding claims, **characterized in that** in step h) the first differentiation factor is CHIR99021.

7. Process according to one of the preceding claims, **characterized in that** in step j) the second differentiation factor is IWP-2.

8. Process according to one of the preceding claims, **characterized in that** in at least one of step h) the third medium contains B27 supplement without insulin, of step i) the fourth medium contains B27 supplement without insulin, of step j) the fifth medium contains B27 supplement without insulin, of step k) the sixth medium contains B27 supplement without insulin, and of step 1) the seventh medium contains B27 supplement containing insulin.

9. Process according to one of the preceding claims, **characterized in that** after at least 1 day subsequent to step h) at least one test compound is added to the medium and, subsequent to incubation, the organoid is analysed and compared to organoids treated in parallel but without the addition of the test compound.

10. Process according to one of the preceding claims, **characterized by** dissolving the hydrogel after one of the steps 1) or m).

11. Process according to one of the preceding claims, **characterized by** adding a test compound to at least one medium of the process and analysing the cardiac organoid in comparison to a cardiac organoid produced in a parallel process performed without the addition of the test compound.

12. Cardiac organoid obtainable by a process according to one of the preceding claims, **characterized in that** the organoid has an inner cavity surrounded by a first layer comprising endothelial cells, which first layer is at least in part surrounded by a second layer comprising cardiomyocytes, which second layer is at least in part surrounded by a third layer comprising cardiomyocytes and epicardial cells, which third layer is at least in part surrounded by a fourth layer comprising fibroblast cells.

13. Cardiac organoid according to claim 12, **characterized in that** the first layer completely surrounds the cavity and is circumferentially closed, that the second layer surrounds the first layer only partially, and that the third layer only surrounds the second layer and that the fourth layer only surrounds the third layer.

14. Cardiac organoid according to one of claims 12 to 13, **characterized in that** it generally has a sphere shape, in which the outer surface is formed by a portion of the first layer, a portion of the second layer and the entire third and/or fourth layer.

15. Cardiac organoid according to one of claims 12 to 14, **characterized in that** it has an outer diameter in the range from 1.2 to 2.5 mm.

16. Use of a cardiac organoid according to one of claims 12 to 15 for use as an implant in the treatment of a patient.

17. Process for analysing the biological effect of at least one test compound on cardiac cells, comprising contacting a cardiac organoid according to one of claims 12 to 15 with the test compound, incubating the cardiac organoid in the presence of the test compound, and analysing the cardiac organoid.
